# EUROPEAN PATENT APPLICATION

(11) **EP 2 149 546 A1**
(43) Date of publication of application: **03.02.2010**
(21) Application number: 08384011.6
(22) Date of filing: 31.07.2008
(51) Int. Cl.: C07C 57/30, C07C 217/74, A61K 31/192, A61K 31/135

(54) **Salts of tramadol and ibuprofen and their crystal form in the treatment of pain**

(71) Applicant: Laboratorios Del. Dr. Esteve, S.A., 08041 Barcelona (ES)
(72) Inventor: Buschmann, Helmut, Heinrich, Dr., 52076 Aachen (Walheim) (DE); Solà Carandell, Lluis, 43893 Altafulla (Tarragona) (ES); Benet Buchholz, Jordi, 43893 Altafulla (Tarragona) (ES); Cerón Bertran, Jordi, Carles, 43005 Tarragona (ES)
(74) Representative: Peters, Hajo

(57) **Abstract**

The present invention relates to salts of tramadol and ibuprofen, their crystal form, processes for preparation of the same and their uses as medicaments, more particularly for the treatment of pain.

## Description

The present invention relates to salts of tramadol and ibuprofen, their crystal form, processes for preparation of the same and their uses as medicaments, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

There are many drugs that are known to be useful in the treatment or management of pain. Opioids are frequently used as analgesics in pain. Derivatives of morphine are indicated for the treatment of moderate to acute pain in human. The analgesic effect is obtained through their action on morphinic receptors, preferably the µ-receptors. Among these derivatives of morphine, may be mentioned morphine, codeine, pethidine, dextropropoxyphenemethadone, lenefopan and others.

One of the morphinic derivatives that has shown very good results when orally administrated, and which is extensively marketed, is Tramadol, also available as a physiologically acceptable salt, particularly as a chlorhydrate. Tramadol, whose chemical name is 2-(dimethylaminomethyl)-1-(3-methoxyphenyl)cyclohexanol, has the following formula :

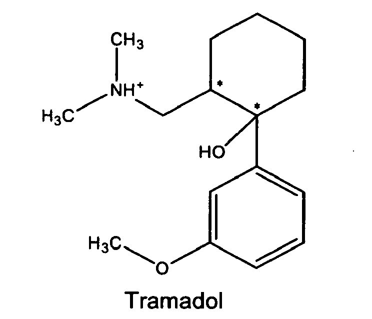

This structure shows two different chiral centers and thus may exist in different diastereoisomers among which the tramadol is the cis-diastereisomer : (1R, 2R), or (1S, 2S), both also known as (+)-tramadol and (-)-tramadol and both of which contribute in different ways to its activity.

From the art it appears that this compound is neither fully opioid-like, nor non-opioid-like. Some studies have demonstrate that tramadol is an opioid agonist, whereas clinical experience indicates that it lacks many of the typical side effects of opioids agonist, for example respiratory depression, constipation or tolerance.

Due to their drawbacks, opioids cannot always be given repeatedly or at higher doses as analgesics to treat pain. The side effects of opioids are known in the art including e.g. J. Jaffe in "Goodman and Gilman's, The Pharmacological Basis of Therapeutics", 8th edition; Gilman et al.; Pergamon Press, New York, 1990, Chapter 22, pages 522-573.

Consequently it has been proposed to combine opioids with other drugs that are not opioid analgesic agents, in order to lower the amount of opioids needed to produce an equivalent degree of analgesia. Among these combinations, the association of tramadol with nonsteroidal anti-inflammatory drugs (NSAIDs) has been reported to be of particular interest (EP-0 546 676).

One interesting NSAIDs to be combined with tramadol is the marketed drug ibuprofen, whose chemical name is 2-[4-(2-methylpropyl)phenyl]propanoic acid, and which is also described as a physiologically acceptable salt. Ibuprofen is showing a chiral center and as such may exist inter alia as racemic mixture or one of two enantiomers (S)-inbuprofen and (R)-ibuprofen. The active enentiomer is (S)-ibuprofen. Nevertheless, most ibuprofen formulations currently marketed are racemic.

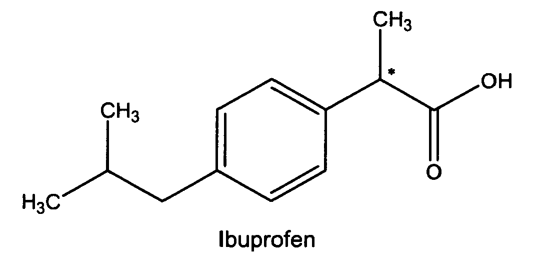

Different formulations have been designed to combine these two active principles (WO-2004/026291), and due to its particular interest, it remains necessary to make new forms of this association available.

The applicant has now found that Tramadol, especially the (+)-Tramadol and (-)-Tramadol, more especially the (R,R)-tramadol, having the opioid activity, and ibuprofen, especially (S)-ibuprofen can be combined to form a mixed-salt.

Thus the object of the present invention is a salt of tramadol and ibuprofen of this formula:

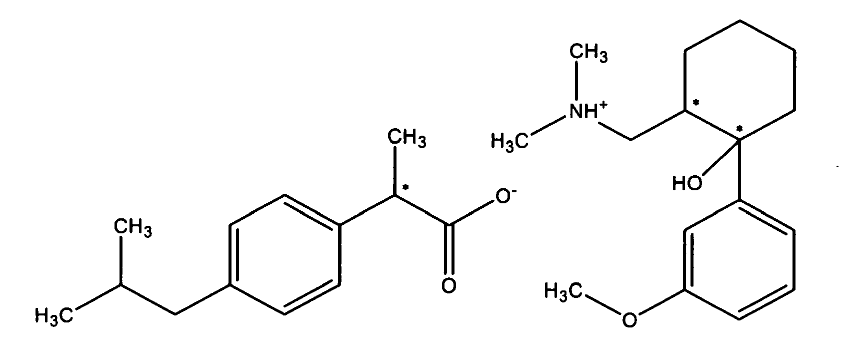

A preferred object of the present invention is a salt of (R,R)-Tramadol and (S)-ibuprofen of this formula:

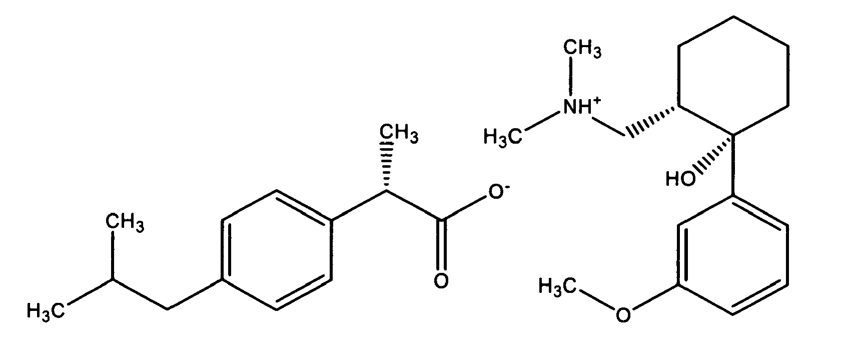

In general each active principle forming part of the salts according to the invention, the tramadol and the ibuprofen, has its own disadvantages when used alone or in the form of the salts known from the art.

Thus, tramadol hydrochloride, which is often used orally, displays a highly bitter taste, which makes the drugs often difficult to swallow and lowers patient compliance. Also - as stated before - the drawbacks associated with opioids is limiting their use, so that they have to be given at lower doses and often less frequent as their use as analgesics to treat pain would normally require.

On the other hand ibuprofen is well-known to be only slightly soluble in water. Less than 1 mg of ibuprofen dissolves in 1 mg of water and this is further limiting its use in pharmaceutical formulations. Even though maybe partly overcome by use of salts like lysine etc. the big majority of these is either not very useful or difficult to formulate, has physiological drawbacks or is only available in very specific formulations. In addition the basic partners of the ibuprofen in the salt are of no pharmaceutical value in themselves only adding - in some cases considerable - molecular weight to the active ingredient thus increasing the overall size of the pharmaceutical formulation without increasing the dosage. As in addition it is well-known that often there are a number of chemical difficulties to be overcome for obtaining salts of ibuprofen or tramadol, there still is a clear need for salts of tramadol or ibuprofen either
- being active in pain or even more active when compared to tramadol base or hydrochloride salt or ibuprofen; or
- being easily obtainable, or
- being easily cristalised, allowing more flexibility in formulating, or
- being highly soluble, especially if compared to ibuprofen, allowing better dissolution rates, especially if dissolving in an aqueous physiological surrounding, or
- having as acidic partner of the tramadol a molecule having a beneficial pharmacological effect in itself, thus allowing for a highly efficient dose/weight relation of the active principle; or
- having as basic partner of the ibuprofen a molecule having a beneficial pharmacological effect in itself, thus allowing for a highly efficient dose/weight relation of the active principle.

Most desirably the salt should combine more than one, most preferably all of these advantages.

The applicant has now found that tramadol and Ibuprofen can be combined to form a well-soluble mixed-salt.

These mixed salts are not only surprisingly easily formed and crystallised they also seem to considerable improve the solubility of ibuprofen. Also this association of the two active principles into the same salt exhibits several further advantages. Being linked as ion and counter-ion, they behave as a single chemical entity, thus facilitating the treatments, formulation, dosage etc. In addition to that, with both tramadol and and ibuprofen being active analgesics these mixed salts are highly useful in the treatment of pain, especially also not losing any activity/weight by the addition of pharmacologically useless counterions. In addition the two active principles are complementing each other in the treatment especially of pain, but possibly also of various other diseases or symptoms. Also the individual dose of the opioid tramadol may be lowered, thus allowing a more frequent treatment, still with an equivalent degree of analgesia. Further, the combination of the two active principles into one unique species advantageously removes the bitter taste of the tramadol hydrochloride, which makes oral application to a patient much easier. Thus, the mixed salts according to the invention do combine a high number of advantages over the state of the art.

The Applicant has further demonstrated the possibility to crystallise said salts. Even though also amorphous salts are also an aspect of the current invention, most preferred are crystalline salts. By that way the physico-chemical properties are improved. The formulation of the mixed salt is even easier with a solid to manipulate and an enhanced stability. The solubility, in particular the solubility of ibuprofen is greatly augmented.

Another advantage is that the association of the two active principle into one unique species seems to allow for a better Pharmacokinetic/Pharmacodynamic (PKPD) including also a better penetration of the blood-brain barrier, which helps in the treatment of pain.

In general in most embodiments in which the salts of tramadol are used (e.g. for the treatment of pain wtc.) these salts would be formulated into a convenient pharmaceutical formulation or a medicament. Accordingly a desirable advantage of a tramadol salt, especially if crystallized, would show improved pharmaceutical properties and features, especially when compared to the free base or tramadol hydrochloride. Thus, the tramadol salt according to the invention, should desirably show at least one, preferably more, of the following features:
- to have a very small particle size, e.g. from 300 µm or lower; or
- to be and/or remain essentially free of agglomerates; or
- to be less or not very hygroscopic; or
- to allow by selection of the counter-ion of the tramadol to help in formulating controlled release or immediate release formulations; or
- to have a high chemical stability; or if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

In one preferred embodiment of the salt according to the invention the tramadol is (-)-tramadol.

In another preferred embodiment of the salt according to the invention the tramadol is (+)-tramadol.

In a further preferred embodiment of the salt according to the invention the ibuprofen is (S)-ibuprofen.

In another further preferred embodiment of the salt according to the invention the salt is selected from
the salt of (-)-tramadol with (S)-ibuprofen; or
the salt of (+)-tramadol with (S)-ibuprofen,
preferably is the salt of (+)-tramadol with (S)-ibuprofen.

In a further preferred embodiment of the salt according to the invention is a salt of (+)-tramadol with (S)-ibuprofen.

In a further preferred embodiment of the salt according to the invention is a salt of (-)-tramadol with (S)-ibuprofen.

The Applicant has further demonstrated the possibility to crystallise said salts. By that way the physico-chemical properties are improved. The formulation of the mixed salt is even easier with a solid to manipulate and an enhanced stability. The solubility, in particular the solubility of the ibuprofen is also greatly augmented.

As the applicant has shown the possibility to crystallise said salts according to the invention a crystalline form of a salt according to the invention, this is a separate, highly intersting aspect of the current invention.

Accordingly, a further aspect of the invention relates to a crystalline form of a salt according to the invention described above.

In a further preferred embodiment of the crystalline form of a salt according to the invention being a salt of (+)-tramadol with (S)-ibuprofen the crystalline form shows a Fourier Transform Infra Red pattern with absorption bands at 3220, 2941, 2926, 2865, 2369, 1702, 1593, 1581, 1481, 1450, 1384 and 1357 cm⁻¹.

In a further preferred embodiment of the crystalline form of a salt according to the invention being a salt of (+)-tramadol with (S)-ibuprofen the crystalline form shows an endothermic peak corresponding to the melting point at an onset at 43°C.

In a further preferred embodiment of a crystalline form of a salt according to the invention being phase 1 of a salt of (-)-tramadol with (S)-ibuprofen the crystalline form shows a Fourier Transform Infra Red pattern with absorption bands at 3022, 2933, 2866, 2233, 1708, 1601, 1580, 1509, 1482, 1455, 1440, 1384 and 1352 cm⁻¹.

In a further preferred embodiment of a crystalline form of a salt according to the invention being phase 1 of a salt of (-)-tramadot with (S)-ibuprofen the crystalline form shows an endothermic peak corresponding to the melting point at an onset at 67°C.

In a further preferred embodiment of a crystalline form of a salt according to the invention being phase 2 of a salt of (-)-tramadot with (S)-ibuprofen the crystalline form shows a Fourier Transform Infra Red pattern with absorption bands at 3022, 2934, 2865, 2237, 1602, 1580, 1482, 1455, 1439, 1391 and 1351 cm⁻¹.

In a further preferred embodiment of a crystalline form of a salt according to the invention being phase 2 of a salt of (-)-tramadol with (S)-ibuprofen the crystalline form shows an endothermic peak corresponding to the melting point at an onset at 65°C.

In a further preferred embodiment of a crystalline form of a salt according to the invention being phase 2 of a salt of (-)-tramadol with (S)-ibuprofen the crystalline form crystallizes as an orthorombic unit with the following dimensions:
- a = 11.73 Å
- b = 25.09 Å
- c = 28.38 Å
- α angle of 90°
- β angle of 90°
- γ angle of 90°

In a further preferred embodiment of a crystalline form being a chloroform solvate of a salt according to the invention the phase 3 of a salt of (-)-tramadol with (S)-ibuprofen has a melting point of between 10 and 15°C.

In a further preferred embodiment of a crystalline form being a chloroform solvate of a salt according to the invention the phase 3 of a salt of (-)-tramadol with (S)-ibuprofen crystallizes as an orthorombic unit with the following dimensions:
- a = 12.04 Å
- b = 17.88 Å
- c = 18.61 Å
- α angle of 90°
- β angle of 90°
- γ angle of 90°

Another embodiment the present invention relates to a process for the production of a salt according to the invention as described above comprising the steps of:
dissolving ibuprofen as a free acid or salt in an organic solvent and either together, before or after dissolving tramadol either as a free base or as a salt in an organic solvent, leading to a mixture in which both active principles are dissolved in one or more organic solvents;
stirring the mixture obtained at a temperature between -20° C and 80°C, evaporating the solvent and/or
filtering, drying and/or purifying the resulting product.

Preferably in the process above
- the organic solvent is selected from acetone, acetonitrile, isobutyl acetate, hepatane, methanol, tetrahydrofuran, isopropanol, ethanol or cyclohexane, preferably is methanol and/or acetone; and/or
- the temperature for stirring the mixture is between -20° C and 30°C; and/or
- the stirring time of the mixture is between 0.25 and 48 hours; and/or
- the solvent is evaporated under high vacuum; and/or
- the ratio of tramadol to ibuprofen is 1 to 1 to 2 to 1, preferably is 1 to 1.

Both parts of the salt are well-known drugs used for a long time worldwide. Due to the therapeutic interest in tramadol in the treatment of pain symptoms and the well-known properties of ibuprofen in this field of medical indication, a further object of the present invention is a medicament containing a tramadol-ibuprofen salt, or its crystalline form according to the invention.

Thus the invention also concerns a medicament comprising at least one salt according to the invention as described above and optionally one or more pharmaceutically acceptable excipients.

A further object of the invention is a pharmaceutical composition **characterized in that** it comprises an efficient amount of the crystalline form of at least one salt according to the invention as described above, in a physiologically acceptable medium.

The medicament according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more of the salts or their crystalline form as defined herein and 40-99 % by weight of one or more auxiliary substances (additives/excipients).

The compositions of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance to be administered during one or several intakes per day.

A further aspect of the invention relates to the use of a salt according to the invention as described above or of a crystalline form of at least one salt according to the invention as described above for the treatment of pain, preferably acute pain, chronic pain, neuropathic pain, hyperalgesia, allodynia or cancer pain, including diabetic neuropathy, fibromyalgia or osteoarthritis.

A related further aspect of the invention is aimed at the use of a salt according to the invention as described above or of a crystalline form of at least one salt according to the invention as described above in the manufacture of a medicament for the treatment of pain, preferably acute pain, chronic pain, neuropathic pain, hyperalgesia, allodynia or cancer pain, including diabetic neuropathy, fibromyalgia or osteoarthritis. Preferably this use is provided for in form of a medicament or a pharmaceutical composition according to the invention as described above.

Another object of the current invention is a method of treatment of pain, preferably acute pain, chronic pain, neuropathic pain, hyperalgesia, allodynia or cancer pain, including diabetic neuropathy, fibromyalgia or osteoarthritis, by providing to a patient in need thereof a sufficient amount of a salt according to the invention as described above or of a crystalline form of at least one salt according to the invention as described above. Preferably a salt according to the invention or its crystalline form according to the invention is provided in physiologically suitable form like e.g. in form of a medicament or a pharmaceutical composition according to the invention as described above.

The present invention is illustrated below with the help of the following figures and examples. These illustrations are given solely by way of example and do not limit the invention.

### Brief description of the figures :

**Figure 1** **: Powder X-Ray diffraction pattern of (+)-tramadol-(*S*)-ibuprofen salt**
**Figure 2****: DSC analysis of (+)-tramadol-(*S*)-ibuprofen salt**
**Figure 3****: TG analysis of (+)-tramadol-(*S*)-ibuprofen salt**
**Figure 4****: Powder X-Ray diffraction pattern of (-)-tramadol-(*S*)-ibuprofen salt, Phase 1**
**Figure 5****: DSC analysis of the (-)-tramadol-(*S*)-ibuprofen salt, Phase 1**
**Figure 6****: TG analysis of (-)-tramadol-(*S*)-ibuprofen salt, Phase 1**
**Figure 7****: Powder X-Ray diffraction pattern of (-)-tramadol-(S)-ibuprofen salt, Phase 2**
**Figure 8****: DSC analysis of (-)-tramadol-(S)-ibuprofen salt, Phase 2**
**Figure 9****: TG analysis of (-)-tramadol-(S)-ibuprofen salt, Phase 2**
**Figure 10****: Structure of (-)-tramadol-(S)-ibuprofen salt, Phase 2 obtained by SCXRD analysis**
**Figure 11****: Structure of (-)-tramadol-(S)-ibuprofen salt, Phase 3 (chloroform solvate) obtained by SCXRD analysis showing three molecules of chloroform in the unit cell**

### EXAMPLE

### Example 1: Preparation of (+)-Tramadol-(S)-ibuprofen salt

### Preparation 1a:

A solution of (S)-ibuprofen (42 mg, 0.20 mmol) in 1 mL of methanol was added to a stirred solution of (+)-tramadol (53 mg, 0.20 mmol) in 1 mL of methanol. The resulting solution was stirred at room temperature for 30 minutes and the solvent was evaporated under vacuum rendering an oil. The oil was dissolved in 0.5 mL of THF and crystallized by vapour diffusion of n-pentane at -17°C. After 5 days a white solid crystallized, which was filtered off and dried under vacuum (10 mm Hg) at 40°C for 4 hours to give the (+)-tramadol-(S)-ibuprofen salt.

### Preparation 1b:

A solution of (S)-ibuprofen (781 mg, 3.79 mmol) in 3 mL of methanol was added to a stirred solution of (+)-tramadol (997 mg, 3.79 mmol) in 3 mL of acetone. The resulting solution was cooled down to -17°C and seed crystals of (+)-tramadol-(S)-ibuprofen salt were added. The mixture was kept at -17°C for 24 hours and a white solid precipitated. The resulting suspension was filtered off, washed with isopropanol and dried under vacuum (10 mm Hg) at 40°C for 6 hours to give the (+)-tramadol-(S)-ibuprofen salt.

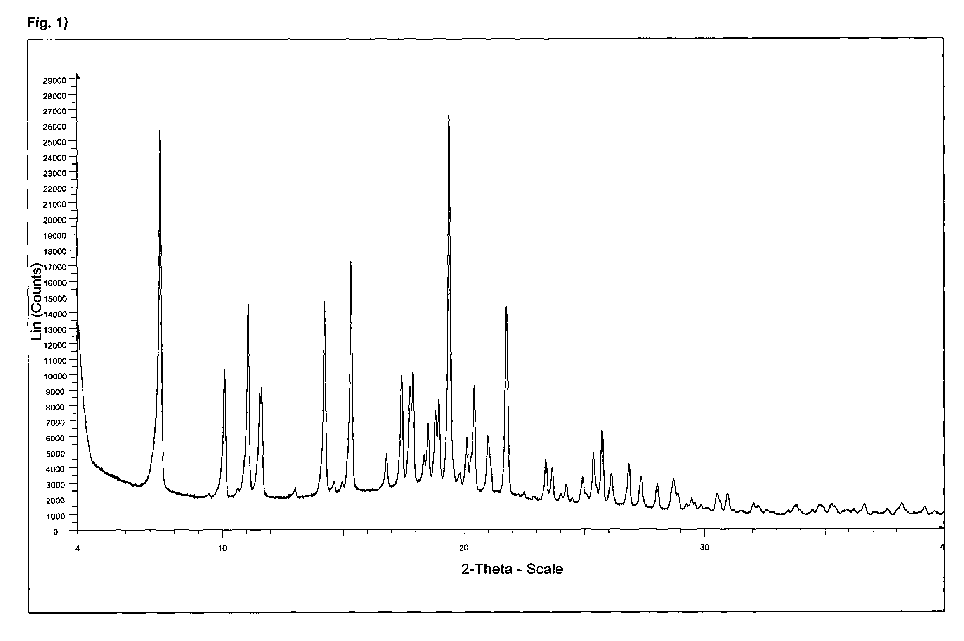

This product was fully characterized by Powder X-Ray diffraction, ¹H-NMR, FTIR, and melting point DSCA and TGA (see figures 1 to 3).

### Powder X-Ray diffraction pattern of (+)-tramadol-(S)-ibuprofen salt (XRPD) (Fig. 1)

Powder diffraction patterns were acquired on a D8 Advance Series 2Theta/Theta powder diffraction system using Cu_{Kα}-radiation in transmission geometry. The system is equipped with a V 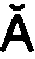 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0. (see figure 1).

**Table 1: List of selected peaks obtained by powder X-Ray diffraction of (+)-tramadol-(S)-ibuprofen salt**

| **Angle 2θ'** | **d-Value (Å)** | **Relative Intensity %** | **Angle 2θ'** | **d-Value (Å)** | **Relative Intensity %** |
|---|---|---|---|---|---|
| 7.424 | 11.89760 | 92.4 | 18.514 | 4.78846 | 20.7 |
| 10.075 | 8.77276 | 33.2 | 18.820 | 4.71130 | 24.1 |
| 10.610 | 8.33109 | 2.5 | 18.963 | 4.67618 | 26.9 |
| 11.056 | 7.99603 | 50.1 | 19.409 | 4.56969 | 100.0 |
| 11.537 | 7.66373 | 27.6 | 19.820 | 4.47592 | 8.1 |
| 11.613 | 7.61423 | 28.7 | 20.119 | 4.40997 | 17.4 |
| 12.995 | 6.80726 | 3.2 | 20.425 | 4.34473 | 30.7 |
| 14.225 | 6.22127 | 51.3 | 20.999 | 4.22710 | 18.0 |
| 14.604 | 6.06059 | 5.2 | 21.092 | 4.20876 | 13.2 |
| 14.933 | 5.92792 | 5.1 | 21.777 | 4.07790 | 51.4 |
| 15.326 | 5.77671 | 61.6 | 22.502 | 3.94809 | 3.9 |
| 16.783 | 5.27837 | 12.6 | 22.901 | 3.88013 | 2.7 |
| 17.423 | 5.08594 | 32.9 | 23.400 | 3.79857 | 12.3 |
| 17.768 | 4.98784 | 30.0 | 23.655 | 3.75822 | 10.2 |
| 17.890 | 4.95403 | 33.6 | 24.038 | 3.69912 | 3.6 |
| 18.340 | 4.83353 | 12.6 | 24.248 | 3.66763 | 5.8 |

| | | | | | |
|---|---|---|---|---|---|
| ¹The 2θ values were obtained using copper radiation (Cu_{Kα1} 1.54060Å) | | | | | |

### ¹H-NMR spectrum of the (+)-tramadol-(S)-ibuprofen salt

Proton nuclear magnetic resonance analyses were recorded in deuterated methanol (MeOH-*d4*) in a Bruker Avance 400 Ultrashield NMR spectrometer, equipped with a z-gradient 5 mm BBO (Broadband Observe) probe. Spectra were acquired solving 2 - 10 mg of sample in 0.6 mL of deuterated solvent.

¹H NMR spectrum, (in D4-methanol at 400 MHz) shows peaks at 0.88 (d, *J* = 7 Hz, 6H), 1.40 (d, *J* = 7 Hz, 3H), 1.44-1.95 (m, 9H), 2.14 (m, 1H), 2.41 - 2.49 (m, 9H), 2.79 (dd, *J* = 9 Hz, *J* = 13 Hz, 1 H), 3.57 (q, *J* = 7 Hz, 1 H), 3.80 (s, 3H), 6.81 (ddd, *J* = 1 Hz, *J* = 3 Hz, *J* = 8 Hz, 1 H), 7.02 - 7.08 (m, 3H), 7.10 (m, 1H), 7.25 (d, *J* = 8 Hz, 2H), 7.28 (t, *J* = 8 Hz, 1 H)

### FT-IR spectrum of the (+)-tramadol-(S)-ibuprofen salt

The FTIR spectra (ATR) of the (+)-tramadol-(S)-ibuprofen salt were recorded using a Bruker Tensor 27, equipped with a MKII golden gate single reflection ATR system, a mid-infrared source as the excitation source and a DTGS detector. The spectra were acquired in 32 scans at a resolution of 4 cm⁻¹.

The sample (KBr pellets) of (+)-tramadol-(S)-ibuprofen salt shows a Fourier Transform Infra Red spectrum (ATR) with absorption bands υₘₐₓ at 3220, 2941, 2926, 2865, 2369, 1702, 1593, 1581, 1481, 1450, 1384 and 1357 cm⁻¹.

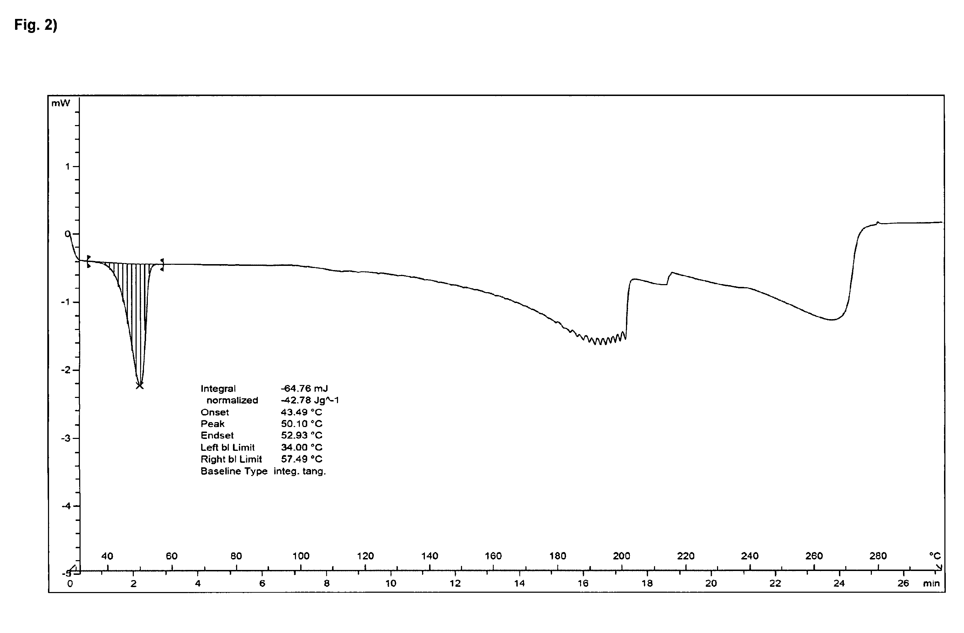

### DSC analysis of the (+)-tramadol-(S)-ibuprofen salt (see Fig. 2)

DSC analyses were recorded in a Mettler Toledo DSC822e. Samples of 1-2 mg were weighted into 40 µL aluminium crucibles with a pinhole lid, and were heated, under nitrogen (50 mL/min), at 10°C/min from 30 to 300°C.

The endothermic peak of the (+)-tramadol-(S)-ibuprofen salt measured was corresponding to the melting point with an onset at 43°C, see figure 2.

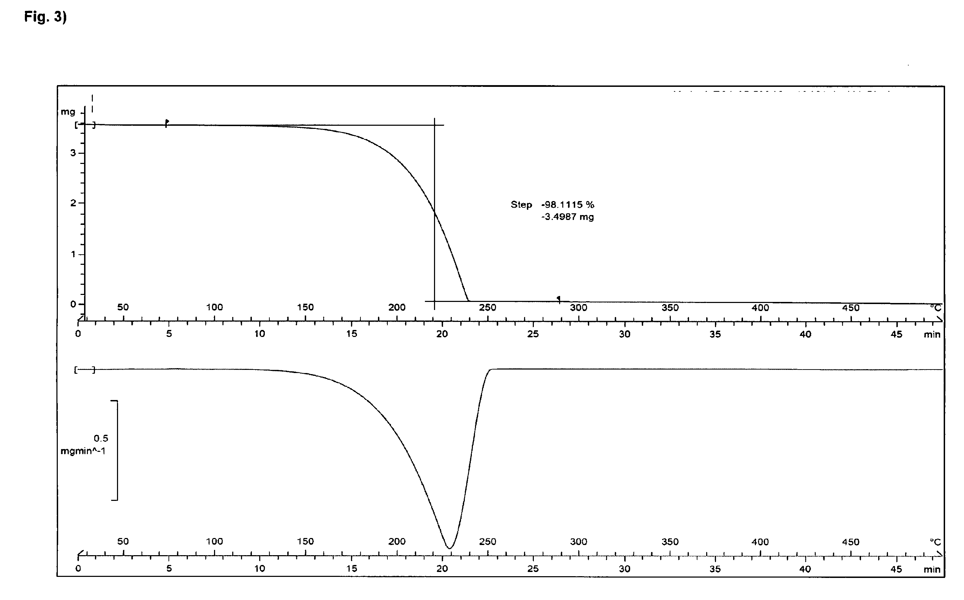

### TG analysis of the (+)-tramadol-(S)-ibuprofen salt (see Fig. 3)

Thermogravimetric analyses were recorded in a Mettler Toledo SDTA851e. Samples of 3 - 4 mg were weighted into 40 µL aluminium crucibles with a pinhole lid, and heated at 10°C/min from 30 to 500°C, under nitrogen (80 mL/min).

The TG analysis of the (+)-tramadol-(S)-ibuprofen salt according to the invention shows weight loss beginning at 130°C due to decomposition (see figure 3).

### Example 2: Preparation of (-)-Tramadol-(S)-ibuprofen salt, Phase 1

### Preparation:

A solution of (*S*)-ibuprofen (46 mg, 0.223 mmol) in 1 mL of methanol was added to a stirred solution of (-)-tramadol (59 mg, 0.224 mmol) in 1 mL of methanol. The resulting solution was stirred at room temperature for 30 minutes and the solvent was evaporated under vacuum rendering an oil. The oil was dissolved in 0.5 mL of *n*-hexane and the solution was allowed to evaporate at -17°C. After 3 weeks a white solid had crystallized, which corresponded to the (-)-tramadol-(*S*)-ibuprofen salt, Phase 1.

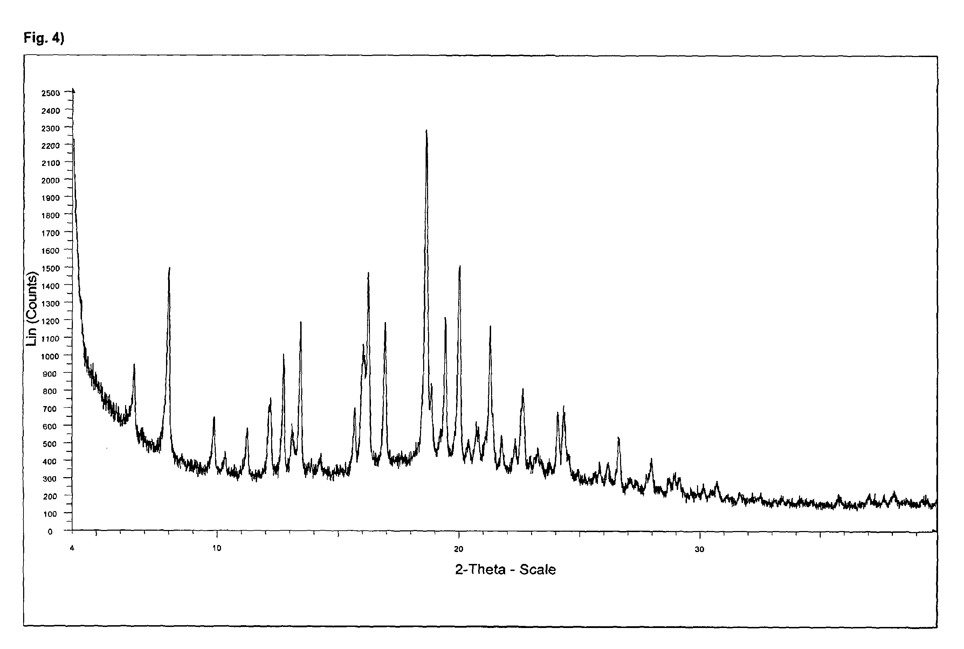

This product was fully characterized by Powder X-Ray diffraction, ¹H-NMR, FTIR, and melting point DSCA and TGA (see figures 4 to 6).

### Powder X-Ray diffraction pattern of (-)-tramadol-(S)-ibuprofen salt, Phase 1 (XRPD) (Fig. 4)

Powder diffraction patterns were acquired on a D8 Advance Series 2Theta/Theta powder diffraction system using Cu_{Kα}-radiation in transmission geometry. The system is equipped with a V 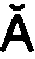 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0 (see figure 4).

**Table 2: List of selected peaks obtained by powder X-Ray diffraction of (-)-tramadol-(S)-ibuprofen salt, Phase 1**

| **Angle 2θ'** | **d-Value (Å)** | **Relative Intensity %** | **Angle 2θ'** | **d-Value (Å)** | **Relative Intensity %** |
|---|---|---|---|---|---|
| 6.548 | 13.48762 | 20.8 | 19.436 | 4.56336 | 44.2 |
| 7.974 | 11.07837 | 55.2 | 20.016 | 4.43237 | 59.3 |
| 9.855 | 8.96764 | 16.0 | 20.404 | 4.34911 | 8.1 |
| 10.322 | 8.56319 | 6.6 | 20.794 | 4.26825 | 12.0 |
| 11.236 | 7.86892 | 14.6 | 21.292 | 4.16958 | 42.5 |
| 12.190 | 7.25507 | 22.5 | 21.768 | 4.07944 | 9.8 |
| 12.734 | 6.94613 | 36.2 | 22.324 | 3.97912 | 9.7 |
| 13.090 | 6.75811 | 15.4 | 22.644 | 3.92371 | 24.8 |
| 13.436 | 6.58488 | 45.6 | 24.097 | 3.69025 | 19.2 |
| 14.251 | 6.20999 | 5.8 | 24.352 | 3.65213 | 19.3 |
| 15.684 | 5.64570 | 19.1 | 25.830 | 3.44639 | 6.8 |
| 16.033 | 5.52338 | 37.7 | 26.178 | 3.40145 | 6.3 |
| 16.243 | 5.45242 | 59.0 | 26.638 | 3.34375 | 14.9 |
| 16.937 | 5.23082 | 43.4 | 27.147 | 3.28215 | 3.4 |
| 18.647 | 4.75475 | 100.0 | 27.383 | 3.25439 | 3.1 |
| 18.854 | 4.70306 | 24.4 | 27.981 | 3.18616 | 10.4 |

| | | | | | |
|---|---|---|---|---|---|
| ¹The 2θ values were obtained using copper radiation (Cu_{Kα1} 1.54060Å) | | | | | |

### ¹H-NMR spectrum of the (-)-tramadol-(S)-ibuprofen salt, Phase 1

Proton nuclear magnetic resonance analyses were recorded in deuterated methanol (MeOH-*d4*) in a Bruker Avance 400 Ultrashield NMR spectrometer, equipped with a z-gradient 5 mm BBO (Broadband Observe) probe. Spectra were acquired solving 2 - 10 mg of sample in 0.6 mL of deuterated solvent.

¹H NMR spectrum, (in D4-methanol at 400 MHz) shows peaks at 0.89 (d, *J* = 6 Hz, 6H), 1.40 (d, *J* = 7 Hz, 3H), 1.44-1.95 (m, 9H), 2.14 (m, 1H), 2.43 (d, *J* = 7 Hz, 2H), 2.45 (dd, *J* = 3 Hz, *J* = 13 Hz, 1H), 2.49 (s, 6H), 2.80 (dd, *J* = 9 Hz, *J* = 13 Hz, 1H), 3.58 (q, *J* = 7 Hz, 1H), 3.80 (s, 3H), 6.82 (ddd, *J* = 1Hz, *J* = 3 Hz, *J* = 8 Hz, 1H), 7.05 (d, *J* = 8 Hz, 3H), 7.10 (m, 1H), 7.24 (d, *J* = 8 Hz, 2H), 7.28 (t, *J* = 8 Hz, 1H).

### FT-IR spectrum of the (-)-tramadol-(S)-ibuprofen salt, Phase 1

The FTIR spectra (ATR) of the (-)-tramadol-(S)-ibuprofen salt, Phase 1 were recorded using a Bruker Tensor 27, equipped with a MKII golden gate single reflection ATR system, a mid-infrared source as the excitation source and a DTGS detector. The spectra were acquired in 32 scans at a resolution of 4 cm⁻¹.

The sample (KBr pellets) of (-)-tramadol-(S)-ibuprofen salt, Phase 1 shows a Fourier Transform Infra Red spectrum (ATR) with absorption bands υₘₐₓ at 3022, 2933, 2866, 2233, 1708, 1601, 1580, 1509, 1482, 1455, 1440, 1384 and 1352 cm⁻¹.

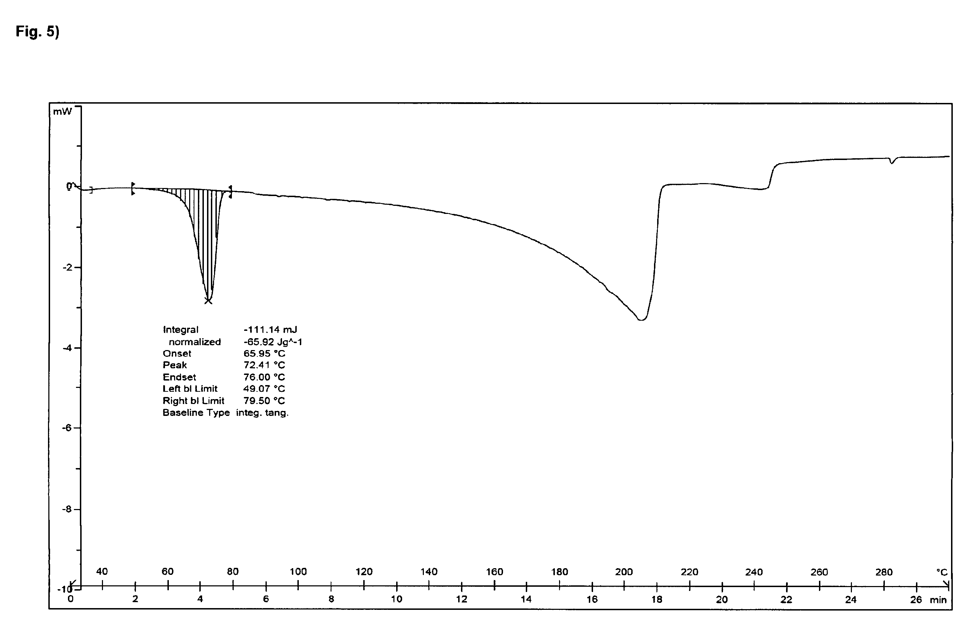

### DSC analysis of the (-)-tramadol-(S)-ibuprofen salt, Phase 1 (see Fig. 5)

DSC analyses were recorded in a Mettler Toledo DSC822e. Samples of 1-2 mg were weighted into 40 µL aluminium crucibles with a pinhole lid, and were heated, under nitrogen (50 mUmin), at 10°C/min from 30 to 300°C.

The endothermic peak of the (-)-tramadol-(S)-ibuprofen salt, phase 1 measured was corresponding to the melting point with an onset at 67°C, see figure 5.

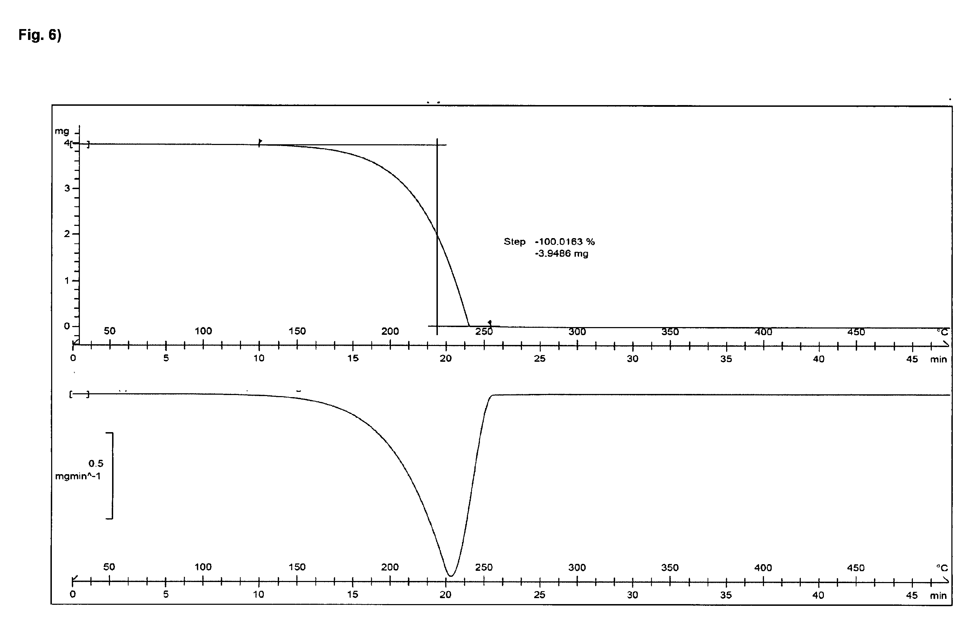

### TG analysis of the (-)-tramadol-(S)-ibuprofen salt, Phase 1 (see Fig. 6)

Thermogravimetric analyses were recorded in a Mettler Toledo SDTA851e. Samples of 3 - 4 mg were weighted into 40 µL aluminium crucibles with a pinhole lid, and heated at 101C/min from 30 to 5001C, under nitrogen (80 mL/min).

The TG analysis of the (-)-tramadol-(S)-ibuprofen salt, Phase 1 according to the invention shows weight loss beginning at 140°C due to decomposition (see figure 6).

### Example 3: Preparation of (-)-Tramadol-(S)-ibuprofen salt, Phase 2

### Preparation:

A solution of (*S*)-ibuprofen (0.88 g, 4.27 mmol) in 5 mL of methanol was added to a stirred solution of (-)-tramadol (1.13 g, 4.29 mmol) in 5 mL of methanol. The resulting solution was stirred at room temperature for 30 minutes and the solvent was evaporated under vacuum rendering an oil. The oil was dissolved in 15 mL of *n*-heptane at 50°C and, after cooling to room temperature, the solution was seeded with 5 - 10 mg of (-)-tramadol-(*S*)-ibuprofen phase 1. After 24 hours a white solid crystallized, which was filtered off, washed with 5 mL of *n*-heptane and dried under vacuum (10 mmHg) at 40°C for 5 hours. The solid obtained corresponded to the (-)-tramadol-(*S*)-ibuprofen salt phase 2 (1.21 g, 60% yield).

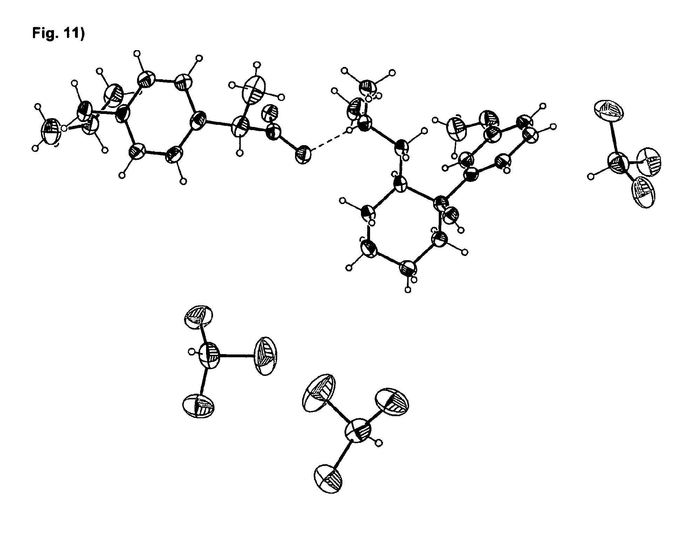

This product was fully characterized by Powder X-Ray diffraction, ¹H-NMR, FTIR, and melting point DSCA and TGA (see figures 11 to 15).

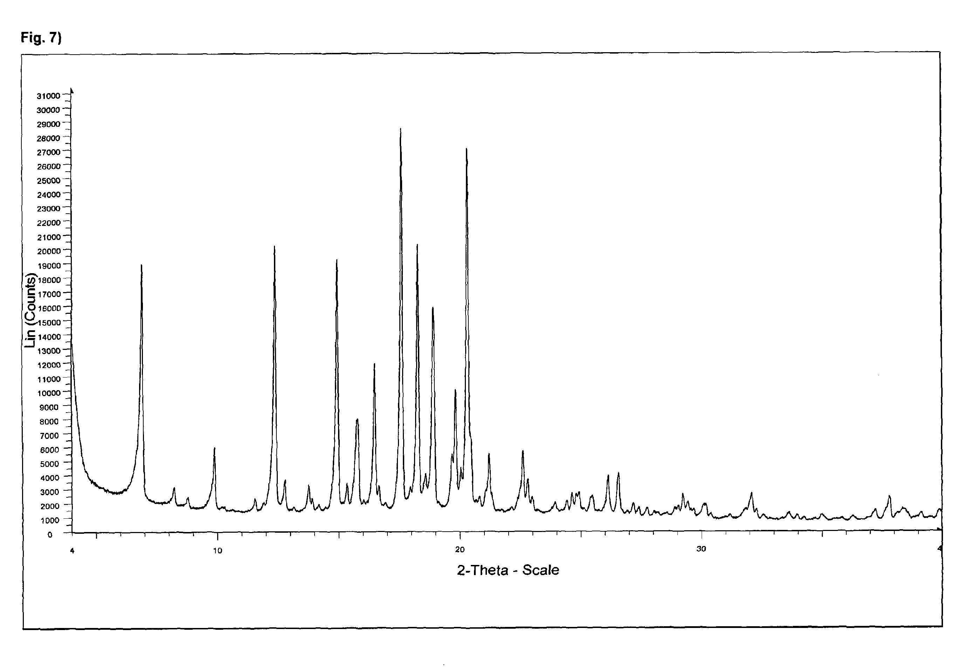

### Powder X-Ray diffraction pattern of (-)-tramadol-(S)-ibuprofen salt, Phase 2 (XRPD) (Fig. 7)

Powder diffraction patterns were acquired on a D8 Advance Series 2Theta/Theta powder diffraction system using Cu_{Kα}-radiation in transmission geometry. The system is equipped with a V 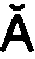 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0 (see figure 7).

**Table 3: List of selected peaks obtained by powder X-Ray diffraction of (-)-tramadol-(S)-ibuprofen salt, Phase 2**

| **Angle 2θ'** | **d-Value (Å)** | **Relative Intensity %** | **Angle 2θ'** | **d-Value (Å)** | **Relative Intensity %** |
|---|---|---|---|---|---|
| 6.870 | 12.85689 | 61.0 | 15.766 | 5.61651 | 24.4 |
| 8.188 | 10.78961 | 4.7 | 16.023 | 5.52684 | 3.8 |
| 8.750 | 10.09783 | 2.6 | 16.479 | 5.37485 | 39.0 |
| 9.847 | 8.97527 | 15.7 | 16.656 | 5.31838 | 7.3 |
| 10.249 | 8.62417 | 1.0 | 16.917 | 5.23697 | 3.1 |
| 10.608 | 8.33335 | 0.5 | 17.602 | 5.03455 | 100.0 |
| 11.533 | 7.66636 | 3.6 | 17.960 | 4.93506 | 7.2 |
| 11.892 | 7.43600 | 2.6 | 18.278 | 4.84991 | 69.9 |
| 12.370 | 7.14975 | 69.1 | 18.575 | 4.77287 | 10.7 |
| 12.758 | 6.93326 | 8.1 | 18.919 | 4.68702 | 53.8 |
| 13.132 | 6.73624 | 1.4 | 19.693 | 4.50443 | 16.0 |
| 13.754 | 6.43333 | 7.3 | 19.826 | 4.47446 | 32.5 |
| 13.890 | 6.37052 | 3.8 | 20.040 | 4.42724 | 12.5 |
| 14.164 | 6.24786 | 2.3 | 20.334 | 4.36390 | 95.0 |
| 14.942 | 5.92435 | 65.7 | 20.814 | 4.26437 | 5.0 |
| 15.342 | 5.77083 | 7.9 | 21.216 | 4.18446 | 16.1 |

| | | | | | |
|---|---|---|---|---|---|
| ¹The 2θ values were obtained using copper radiation (Cu_{Kα1} 1.54060Å) | | | | | |

### ¹H-NMR spectrum of the (-)-tramadol-(S)-ibuprofen salt, Phase 2

Proton nuclear magnetic resonance analyses were recorded in deuterated methanol (MeOH-*d4*) in a Bruker Avance 400 Ultrashield NMR spectrometer, equipped with a z-gradient 5 mm BBO (Broadband Observe) probe. Spectra were acquired solving 2 - 10 mg of sample in 0.6 mL of deuterated solvent.

¹H NMR spectrum, (in D4-methanol at 400 MHz) shows peaks at 0.88 (d, *J* = 7 Hz, 6H), 1.40 (d, *J* = 7 Hz, 3H), 1.44-1.94 (m, 9H), 2.14 (m, 1H), 2.43 (d, *J* = 7 Hz, 2H), 2.43 (dd, *J* = 3 Hz, *J* = 13 Hz, 1H), 2.47 (s, 6H), 2.79 (dd, *J* = 9 Hz, *J* = 13 Hz, 1H), 3.58 (q, *J* = 7 Hz, 1H), 3.80 (s, 3H), 6.81 (ddd, *J* = 1 Hz, *J* = 3 Hz, *J* = 8 Hz, 1H), 7.05 (d, *J* = 8 Hz, 3H), 7.10 (m, 1H), 7.25 (d, *J* = 8 Hz, 2H), 7.28 (t, *J* = 8 Hz, 1H).

### FT-IR spectrum of the (-)-tramadol-(S)-ibuprofen salt, Phase 2

The FTIR spectra (ATR) of the (-)-tramadol-(S)-ibuprofen salt, Phase 2 were recorded using a Bruker Tensor 27, equipped with a MKII golden gate single reflection ATR system, a mid-infrared source as the excitation source and a DTGS detector. The spectra were acquired in 32 scans at a resolution of 4 cm⁻¹.

The sample (KBr pellets) of (-)-tramadol-(S)-ibuprofen salt, Phase 2 shows a Fourier Transform Infra Red spectrum (ATR) with absorption bands υₘₐₓ at 3022, 2934, 2865, 2237, 1602, 1580, 1482, 1455, 1439, 1391 and 1351 cm⁻¹.

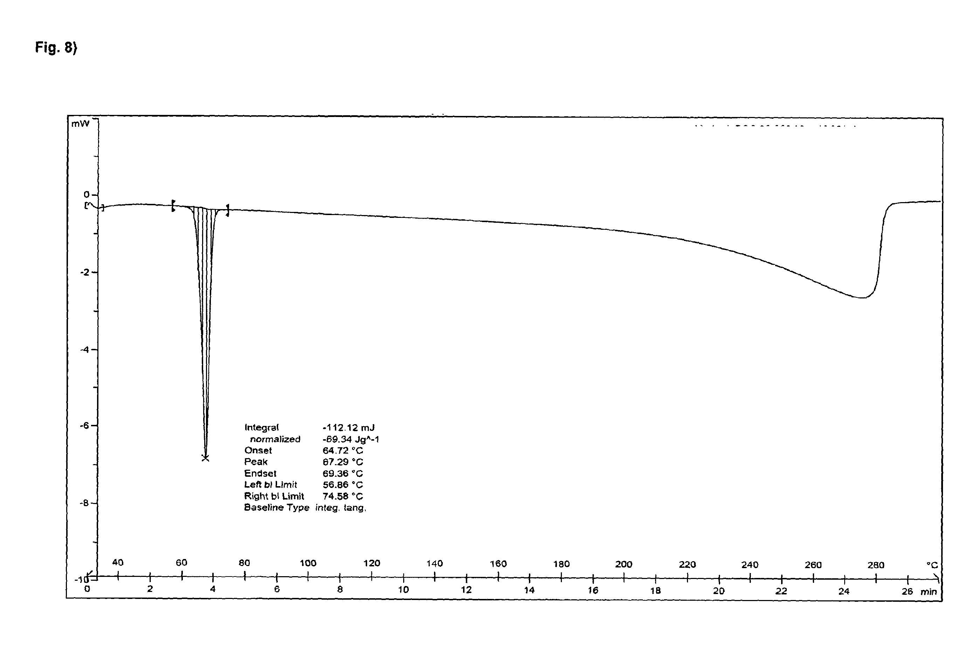

### DSC analysis of the (-)-tramadol-(S)-ibuprofen salt, Phase 2 (see Fig. 8)

DSC analyses were recorded in a Mettler Toledo DSC822e. Samples of 1-2 mg were weighted into 40 µL aluminium crucibles with a pinhole lid, and were heated, under nitrogen (50 mL/min), at 10°C/min from 30 to 300°C.

The endothermic peak of the (-)-tramadol-(S)-ibuprofen salt, phase 2 measured was corresponding to the melting point with an onset at 65°C, see figure 8.

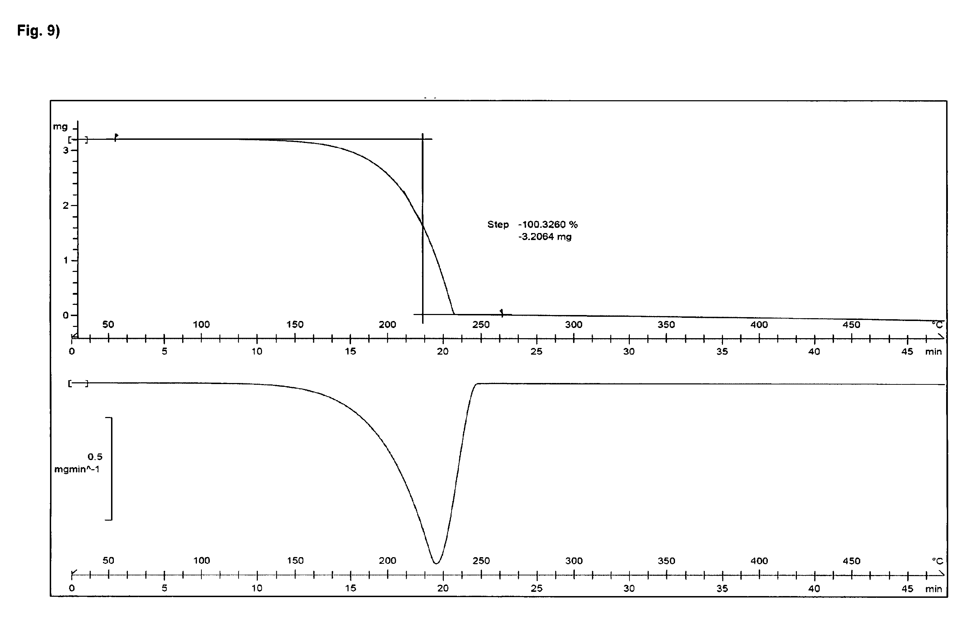

### TG analysis of the (-)-tramadol-(S)-ibuprofen salt, Phase 2 (see Fig. 9)

Thermogravimetric analyses were recorded in a Mettler Toledo SDTA851e. Samples of 3 - 4 mg were weighted into 40 µL aluminium crucibles with a pinhole lid, and heated at 10°C/min from 30 to 500°C, under nitrogen (80 mL/min).

The TG analysis of the (-)-tramadol-(S)-ibuprofen salt, Phase 2 according to the invention shows weight loss beginning at 130°C due to decomposition (see figure 9).

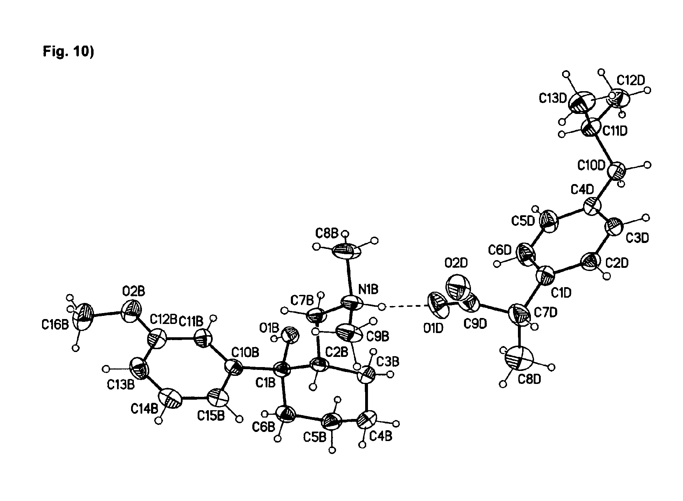

### Single Crystal XRD analysis of a single crystal of the (-)-tramadol-(S)-ibuprofen salt, Phase 2 (see Fig. 10)

The crystal structure was determined from single crystal X-ray diffraction data. The measured crystal obtained from the preparation according to example 3 was selected using a Zeiss stereomicroscope using polarized light and prepared under inert conditions immersed in perfluoropolyether as protecting oil for manipulation. Crystal structure determination was carried out using a Bruker-Nonius diffractometer equipped with a APPEX 2 4K CCD area detector, a FR591 rotating anode with Mo_{Kα} radiation, Montel mirrors as monochromator and a Kryoflex low temperature device (T = 100 K). Fullsphere data collection omega and phi scans. Programs used: Data collection Apex2 V. 1.0-22 (Bruker-Nonius 2004), data reduction Saint + Version 6.22 (Bruker-Nonius 2001) and absorption correction SADABS V. 2.10 (2003). Crystal structure solution was achieved using direct methods as implemented in SHELXTL Version 6.10 (Sheldrick, Universtität Göttingen (Germany), 2000) and visualized using XP program. Missing atoms were subsequently located from difference Fourier synthesis and added to the atom list. Least-squares refinement on F₀² using all measured intensities was carried out using the program SHELXTL Version 6.10 (Sheldrick, Universtität Göttingen (Germany), 2000). All non hydrogen atoms were refined including anisotropic displacement parameters.

**Table 4a: Most relevant structural data of the SCXRD analysis of (-)-tramadol-(S)-ibuprofen salt, Phase 2**

| Crystal system | Orthorombic |
|---|---|
| Space group | *P*2₁2₁2₁ |
| a (Å) | 11.7338(3) |
| b (Å) | 25.0910(7) |
| c (Å) | 28.3826(8) |
| α (°) | 90 |
| β (°) | 90 |
| γ (°) | 90 |
| Z | 4 |
| Volume (Å³) | 8356.2(4) |

**Table 4b: Complete Structural Data of the SCXRD analysis of (-)-tramadol-(S)-ibuprofen salt, Phase 2**

| | | |
|---|---|---|
| Identification code | p12ibphep_0m | |
| Empirical formula | C87 H130 N3 012.50 | |
| Formula weight | 1417.94 | |
| Temperature | 100(2) K | |
| Wavelength | 0.71073 Å | |
| Crystal system | Orthorhombic | |
| Space group | *P*2₁2₁2₁ | |
| Unit cell dimensions | a = 11.7338(3) Å | α = 90° |
| | b = 25.0910(7) Å | β = 90° |
| | c = 28.3826(8) Å | γ = 90° |
| Volume | 8356.2(4) Å³ | |
| Z | 4 | |
| Density (calculated) | 1.127 Mg/m³ | |
| Absorption coefficient | 0.074 mm⁻¹ | |
| F(000) | 3092 | |
| Crystal size | 0.20 x 0.20 x 0.06 mm³ | |
| Theta range for data collection | 2.77 to 33.51°. | |
| Index ranges | -15 ≤h ≤18, -33 ≤k ≤38, -28 ≤l ≤43 | |
| Reflections collected | 92108 | |
| Independent reflections | 31594 [R(int) = 0.0490] | |
| Completeness to theta = 33.51° | 96.4 % | |
| Absorption correction | SADABS (Bruker-Nonius) | |
| Max. and min. transmission | 0.9956 and 0.9853 | |
| Refinement method | Full-matrix least-squares on F² | |
| Data / restraints / parameters | 31594 / 2 / 1144 | |
| Goodness-of-fit on F² | 1.027 | |
| Final R indices [I>2sigma(I)] | R1 = 0.0669, wR2 = 0.1545 | |
| R indices (all data) | R1 = 0.1106, wR2 = 0.1782 | |
| Absolute structure parameter | 0.2(6) | |
| Largest diff. peak and hole | 0.520 and -0.316 e.Å⁻³ | |

### Example 4: Preparation of (-)-Tramadol-(S)-ibuprofen salt, Phase 3 (chloroform solvate)

### Preparation:

A solution of (S)-ibuprofen (46 mg, 0.22 mmol) in 1 mL of methanol was added to a stirred solution of (-)-tramadol (59 mg, 0.22 mmol) in 1 mL of methanol. The resulting solution was stirred at room temperature for 30 minutes and the solvent was evaporated under vacuum rendering an oil. The oil was dissolved in 0.5 mL of chloroform and the solution was allowed to evaporate at -17°C. After 2 weeks a white solid had crystallized, which corresponded to the chloroform solvate of (-)-tramadol-(S)-ibuprofen salt.

### Melting Point:

The (-)-Tramadol-(*S*)-ibuprofen salt, Phase 3 (chlorofom solvate) showed a melting point of 10 - 15 °C.

### ¹H-NMR spectrum of the (-)-tramadol-(S)-ibuprofen salt, Phase 3 (chlorofom solvate)

Proton nuclear magnetic resonance analyses were recorded in deuterated methanol (MeOH-*d4*) in a Bruker Avance 400 Ultrashield NMR spectrometer, equipped with a z-gradient 5 mm BBO (Broadband Observe) probe. Spectra were acquired solving 2 - 10 mg of sample in 0.6 mL of deuterated solvent.

¹H NMR spectrum, (in D4-methanol at 400 MHz) shows peaks at 0.89 (d, *J* = 7 Hz, 6H), 1.40 (d, *J* = 7 Hz, 3H), 1.43 - 1.94 (m, 9H), 2.14 (m, 1H), 2.40 - 2.50 (m, 9H), 2.81 (dd, *J* = 9 Hz, *J* = 13 Hz, 1H), 3.56 (q, *J* = 7 Hz, 1H), 3.80 (s, 3H), 6.81 (dd, *J* = 3 Hz, *J* = 8 Hz, 1H), 7.02 - 7.13 (m, 4H), 7.25 (d, *J* = 8 Hz, 2H), 7.28 (t, *J* = 8 Hz, 1H), 7.89 (CHCl₃).

### Single Crystal XRD analysis of a single crystal of the (-)-tramadol-(S)-ibuprofen salt, Phase 3 (Chloroform Solvate) (see Fig. 11)

The crystal structure was determined from single crystal X-ray diffraction data. The measured crystal obtained from the preparation according to example 4 was selected using a Zeiss stereomicroscope using polarized light and prepared under inert conditions immersed in perfluoropolyether as protecting oil for manipulation. Crystal structure determination was carried out using a Bruker-Nonius diffractometer equipped with a APPEX 2 4K CCD area detector, a FR591 rotating anode with Mo_{Kα} radiation, Montel mirrors as monochromator and a Kryoflex low temperature device (T = 100 K). Fullsphere data collection omega and phi scans. Programs used: Data collection Apex2 V. 1.0-22 (Bruker-Nonius 2004), data reduction Saint + Version 6.22 (Bruker-Nonius 2001) and absorption correction SADABS V. 2.10 (2003). Crystal structure solution was achieved using direct methods as implemented in SHELXTL Version 6.10 (Sheldrick, Universtität Göttingen (Germany), 2000) and visualized using XP program. Missing atoms were subsequently located from difference Fourier synthesis and added to the atom list. Least-squares refinement on F₀² using all measured intensities was carried out using the program SHELXTL Version 6.10 (Sheldrick, Universtität Göttingen (Germany), 2000). All non hydrogen atoms were refined including anisotropic displacement parameters.

**Table 5a: Most relevant structural data of the SCXRD analysis of (-)-tramadol-(S)-ibuprofen salt, Phase 3 (Chloroform Solvate)**

| Crystal system | Orthorombic |
|---|---|
| Space group: | *P*2₁2₁2₁ |
| a (Å) | 12.0380(6) |
| b (Å) | 17.8764(8) |
| c (Å) | 18.6124(7) |
| □(°) | 90 |
| □(°) | 90 |
| □(°) | 90 |
| Z | 4 |
| Volume (Å³) | 4005.3(3) |

**Table 5b: Complete Structural Data of the SCXRD analysis of (-)-tramadol-(S)-ibuprofen salt, Phase 3 (Chloroform solvate)**

| | | |
|---|---|---|
| Identification code | p012ibpclf_0m | |
| Empirical formula | C32 H46 Cl9 N O4 | |
| Formula weight | 827.75 | |
| Temperature | 100(2) K | |
| Wavelength | 0.71073 Å | |
| Crystal system | Orthorhombic | |
| Space group | *P*2₁2₁2₁ | |
| Unit cell dimensions | a = 12.0380(6) Å | α = 90° |
| | b = 17.8764(8) Å | β = 90° |
| | c = 18.6124(7) Å | γ = 90° |
| Volume | 4005.3(3) Å³ | |
| Z | 4 | |
| Density (calculated) | 1.373 Mg/m³ | |
| Absorption coefficient | 0.664 mm⁻¹ | |
| F(000) | 1720 | |
| Crystal size | 0.10 x 0.05 x 0.02 mm³ | |
| Theta range for data collection | 2.53 to 33.79°. | |
| Index ranges | -10 ≤h ≤18, -27 ≤k ≤27, -29 ≤l ≤29 | |
| Reflections collected | 75940 | |
| Independent reflections | 15782 [R(int) = 0.0793] | |
| Completeness to theta = 33.79° | 98.3 % | |
| Absorption correction | SADABS (Bruker-Nonius) | |
| Max. and min. transmission | 0.9868 and 0.9366 | |
| Refinement method | Full-matrix least-squares on F² | |
| Data / restraints / parameters | 15782 / 0 / 470 | |
| Goodness-of-fit on F² | 1.023 | |
| Final R indices [I>2sigma(I)] | R1 = 0.0665, wR2 = 0.1701 | |
| R indices (all data) | R1 = 0.1124, wR2 = 0.2008 | |
| Absolute structure parameter | 0.00(6) | |
| Largest diff. peak and hole | 0.759 and -0.424 e.Å⁻³ | |

## Claims

1. A salt of tramadol with ibuprofen.

2. The salt according to claim 1 in which the ibuprofen is (S)-ibuprofen.

3. The salt according to claim 1 of (+)-tramadol with (S)-ibuprofen

4. The salt according to claim 1 of (-)-tramadol with (S)-ibuprofen

5. Crystalline form of a salt according to any of claims 1 to 4.

6. Crystalline form of a salt according to claim 3, **characterized in that** it shows a Fourier Transform Infra Red pattern with absorption bands at 3220, 2941, 2926, 2865, 2369, 1702, 1593, 1581, 1481, 1450, 1384 and 1357 cm⁻¹.

7. Crystalline form of a salt according to claim 3, **characterized in that** the endothermic peak corresponding to the melting point has an onset at 43°C.

8. Crystalline form phase 1 of a salt according to claim 4, **characterized in that** it shows a Fourier Transform Infra Red pattern with absorption bands at 3022, 2933, 2866, 2233, 1708, 1601, 1580, 1509, 1482, 1455, 1440, 1384 and 1352 cm⁻¹.

9. Crystalline form phase 1 of a salt according to claim 4, **characterized in that** the endothermic peak corresponding to the melting point has an onset at 67°C.

10. Crystalline phase 2 of a salt according to claim 4, **characterized in that** it shows a Fourier Transform Infra Red pattern with absorption bands at 3022, 2934, 2865, 2237, 1602, 1580, 1482, 1455, 1439, 1391 and 1351 cm⁻¹.

11. Crystalline form phase 2 of a salt according to claim 4, **characterized in that** it crystallizes as an orthorombic unit with the following dimensions :
• a=11.73 Å
• b = 25.09 Å
• c = 28.38 Å
• α angle of 90°
• β angle of 90°
• γ angle of 90°

12. Crystalline form phase 2 of a salt according to claim 4, **characterized in that** the endothermic peak corresponding to the melting point has an onset at 65 °C.

13. Crystalline form phase 3, being a chloroform solvate of a salt according to claim 4, **characterized in that** it has a melting point of between 10 and 15°C.

14. Crystalline form phase 3, being a chloroform solvate of a salt according to claim 4, **characterized in that** it crystallizes as an orthorombic unit with the following dimensions:
• a = 12.04 Å
• b = 17.88 Å
• c = 18.61 Å
• α angle of 90°
• β angle of 90°
• γ angle of 90°

15. Process for the production of a salt according to any of claims 1 to 4 comprising the steps of:
• dissolving ibuprofen as a free acid or salt in an organic solvent and either together, before or after dissolving tramadol either as a free base or as a salt in an organic solvent, leading to a mixture in which both active princuples are dissolved in one or more organic solvents;
• stirring the mixture obtained at a temperature between -20° C and 80°C,
• evaporating the solvent and/or
• filtering, drying and/or purifying the resulting product.

16. Process according to claim 15, wherein
• the organic solvent is selected from acetone, acetonitrile, isobutyl acetate, hepatane, methanol, tetrahydrofuran, isopropanol, ethanol or cyclohexane, preferably is methanol and/or acetone; and/or
• the temperature for stirring the moxtur is between -20° C and 30°C; and/or
• the stirring time of the mixture is between 0,25 and 48 hours; and/or
• the solvent is evaporated under high vacuum; and/or
• the ratio of tramadol to ibuprofen is 1 to 1 to 2 to 1, preferably is 1 to 1.

17. Medicament comprising at least one salt according to any of claims 1 to 4 and optionally one or more pharmaceutically acceptable excipients.

18. Pharmaceutical composition **characterized in that** it comprises a therapeutically effective amount of the salt according to any one of claims 1 to 4 or the crystalline form of a salt according to any one of claims 5 to 14, in a physiologically acceptable medium.

19. Use of a salt according to any one of claims 1 to 4 or the crystalline form of a salt according to any one of claims 5 to 14 for the treatment of pain, preferably acute pain, chronic pain, neuropathic pain, hyperalgesia, allodynia or cancer pain, including diabetic neuropathy and osteoarthritis.
